# FASCICULE DE BREVET EUROPEEN

(11) **EP 1 996 200 B1**
(45) Date de publication et mention de la délivrance du brevet: **08.05.2013**
(21) Numéro de dépôt: 07712456.8
(22) Date de dépôt: 06.03.2007
(51) Int. Cl.: A61K 31/52, A61P 37/00

(54) **UTILISATION DE COMPOSÉS DÉRIVÉS DE L'ADÉNINE POUR LE TRAITEMENT DU LUPUS**
VERWENDUNG VON VERBINDUNGEN AUS ADENIN ZUR BEHANDLUNG VON LUPUS
USE OF ADENINE-DERIVED COMPOUNDS FOR THE TREATMENT OF LUPUS

(30) Priorité: 06.03.2006 FR 0601958
(43) Date de publication de la demande: 03.12.2008
(73) Titulaire: CENTRE NATIONAL DE LA RECHERCHE SCIENTIFIQUE (CNRS), 75016 Paris (FR)
(72) Inventeur: LUGNIER, Claire, Renée, Jeanne, F-67000 Strasbourg (FR); MULLER, Sylviane, Paule, Ghislaine, F-67000 Strasbourg (FR); MONNEAUX, Fanny, Sylvie, Michèle, F-67610 La Wantzenau (FR); BOURGUIGNON, Jean-Jacques, F-67400 Illkirch (FR)
(74) Mandataire: Regimbeau
(86) Numéro de dépôt international: PCT/EP2007/052084
(87) Numéro de publication internationale: WO 2007/101850

(56) Documents cités:
- EP-A1- 1 043 021
- WO-A-2004/014913
- US-A1- 2003 104 974
- US-B1- 6 716 987
- BOICHOT ELISABETH ET AL: "Anti-inflammatory activities of a new series of selective phosphodiesterase 4 inhibitors derived from 9-benzyladenine" JOURNAL OF PHARMACOLOGY AND EXPERIMENTAL THERAPEUTICS, vol. 292, no. 2, février 2000 (2000-02), pages 647-653, XP001106309 ISSN: 0022-3565

## Description

L'invention concerne l'utilisation de composés dérivés de l'adénine, substitués en positions 2 et 9, et éventuellement N(6) de l'adénine, pour la fabrication d'un médicament destiné au traitement du lupus érythémateux disséminé (LED). Les composés peuvent également être utilisés en association avec un deuxième composé utilisé dans le traitement du LED.

Le lupus érythémateux disséminé ou lupus érythémateux systémique (LES) est une affection auto-immune très polymorphe d'origine multigénique. Il représente l'archétype des maladies auto-immunes non spécifiques d'organes. Il touche en moyenne 15-50 cas sur 100000 personnes/an. En France, environ 50000 à 80000 personnes sont touchées. Il survient principalement chez la femme (environ 9 cas sur 10), et certaines populations ethniques semblent prédisposées au développement de cette maladie, notamment les populations antillaises, afro-américaines et hispano-américaines.

Bien que l'origine de la maladie ne soit pas connue, il est clair qu'elle est multifactorielle et différents types de facteurs étiologiques ont été identifiés. Concernant les facteurs génétiques, la susceptibilité à la maladie est clairement d'origine multigénique. En effet, plusieurs gènes impliqués dans une susceptibilité génétique ont pu être mis en évidence, tels que notamment les allèles DR2 et DR3 du système HLA. D'autres gènes non liés au système HLA sont également impliqués.

De plus, des facteurs environnementaux ont été identifiés, tels que les rayons ultraviolets (caractère photosensible de l'éruption lupique) et les hormones sexuelles (femme en période d'activité génitale, rôle de et sur la grossesse).

Le LED est une maladie extrêmement polymorphe et ses symptômes et manifestations cliniques sont donc très variés. Le diagnostic de la maladie est donc souvent difficile. Il ne peut être fait qu'après un interrogatoire minutieux et des examens sanguins. Pour établir le diagnostic, on utilise les critères de l'Association des Rhumatologues Américains (ARA). Quatre de ces critères doivent être présents pour affirmer un diagnostic de lupus. Ils sont présents dans 96% des LED :
1. Éruption sur le visage en aile de papillon
2. Éruption du lupus discoïde
3. Photosensibilité
4. Ulcérations buccales ou nasopharyngées
5. Polyarthrite non érosive
6. Pleurésie ou péricardite
7. Néphropathie : protéinurie > 0,5 g/24 h ou cylindrurie
8. Crises comitiales ou psychose
9. Atteintes hématologiques : Anémie hémolytique ou leucopénie < 4000/mm³ ou lymphopénie < 1500/mm³ ou thrombopénie < 100 000/mm³
10. Anomalie immunologique : présence de cellules LE (cellules de Hargraves) ou anticorps (Ac) anti-ADN natifs ou anticorps anti-Sm ou fausse sérologie syphilitique positive.
11. Présence d'un taux élevé d'Ac antinucléaires.

Parmi les manifestations cliniques du lupus, la néphropathie lupique est une manifestation fréquente (estimée entre 35 et 55 % sur les paramètres biologiques usuels) et représente un des principaux facteurs de pronostic. Elle peut être détectée par la recherche d'une hématurie et/ou leucocyturie avec formation de cylindres, d'une hypertension artérielle, ou le plus souvent d'une protéinurie. La détection d'une néphropathie constitue un tournant conditionnant le pronostic de la maladie. En effet, elle est susceptible d'évoluer vers une insuffisance rénale chronique dans un délai de 5 à 10 ans. Seule la dialyse ou une greffe de rein permet alors la survie des patients.

Sur le plan biologique, le LED se caractérise par :
- un syndrome inflammatoire généralisé lors des poussées lupiques, caractérisé notamment par une sécrétion importante de TNF-α;
- des troubles hématologiques ;
- des anomalies sérologiques dominées par l'existence d'Ac anti-nucléaires (AAN) pouvant comprendre des Ac anti-ADN, anti-histones, anti-nucléosomes, anti-Sm, des anticorps anti-SSA ou anti-SSB, et des Ac anti-ribonucléoprotéines (RNP). Les patients produisent également des Ac dirigés contre les éléments figurés du sang ou les phospholipides ; certains de ces auto-Ac pouvant participer à la formation de complexes immuns circulants ; et
- une hypocomplémentémie liée à la consommation du complément par les complexes immuns (liée à une atteinte rénale grave et se corrigeant lors des rémissions), et/ou à un déficit constitutionnel en C2 ou C4 (prédisposant au LED).

Du fait de l'origine encore inconnue de la maladie, il n'existe actuellement aucun traitement spécifique pour le LED. En fonction de la sévérité de la maladie et des symptômes détectés, différentes alternatives thérapeutiques sont utilisées.

Les lupus quiescents ne justifient qu'une simple surveillance.

Le traitement des formes mineures cutanéor-articulaires repose sur les anti-inflammatoires non stéroidiens (AINS), par exemple l'aspirine (2 à 4g par jour), et les anti-paludéens de synthèse, par exemple l'hydroxychloroquine ou la chloroquine. Les AINS permettent de calmer les symptômes mais présentent des risques digestifs (ulcères de l'estomac), allergiques et rénaux (insuffisance rénale). Le mode d'action des anti-paludéens de synthèse dans le lupus est mal connu, mais leur efficacité est démontrée. L'hydroxychloroquine (Plaquenil®) est généralement employée à la dose de 400 mg/j. L'efficacité est jugée après 3 mois. Cependant, une surveillance ophtalmologique annuelle (vision des couleurs, échelle d'Amsler) est nécessaire pour rechercher d'éventuels signes de toxicité rétinienne, principal effet secondaire de ces composés, qui imposent l'arrêt du traitement. Les autres effets secondaires (ex : neuromyopathie, agranulocytose) sont plus rares.

La persistance de symptômes articulaires peut amener à administrer une corticothérapie douce (inférieure à 10 mg/j de prednisone). A l'inverse, une atteinte cutanée résistante aux anti-paludéens ne constitue pas une indication à la corticothérapie, mais justifie le recours à d'autres thérapeutiques (association d'antipaludéens, thalidomide....).

Le traitement des formes viscérales repose sur la corticothérapie. La prednisone (Cortancyl® par exemple) est le corticoïde de référence. Schématiquement, la posologie employée est de 1 à 1,5 mg/kg/j dans les formes graves (glomérulonéphrite proliférative diffuse, anémie hémolytique) et de 0,5 mg/kg/j dans les sérites. Cependant, la corticothérapie produit des effets secondaires significatifs, et certains d'entre eux doivent être prévenus. En particulier, le rôle de la corticothérapie dans l'accélération de l'athérogénèse impose de prendre en compte ses diverses composantes (HTA, diabète, dyslipidémie, tabagisme...) : une diététique excluant le sodium et restreignant les apports glucidiques est donc conseillée, généralement associée à une supplémentation potassique. L'utilisation préventive des pansements gastriques et le traitement curatif par les anti-H2 ont réduit les complications digestives, surtout présentes en cas d'association avec les AINS. Au plan osseux, l'ostéoporose semble atténuée par l'adjonction quotidienne de vitamine D et de calcium en alternance avec les diphosphonates. Les risques infectieux sont considérablement majorés par la corticothérapie à fortes doses, ce qui justifie le dépistage et le traitement systématique des foyers infectieux latents. En pratique, la corticothérapie d'attaque est prescrite sur une durée de 6 semaines à 3 mois. La régression, progressive, se fait par diminution de 10% de la dose antérieure, tous les 5 à 15 jours. Le sevrage, lorsqu'il est tenté, doit être précédé de l'exploration de l'axe hypothalamo-hypophyso-surrénalien. L'administration de fortes doses de corticoïdes en bolus intraveineux est employée dans le traitement des poussées graves, notamment rénales et neurologiques. Les patients reçoivent 500mg-1g de méthylprednisolone (Solumédrol® par exemple) en 3h pendant 3 jours consécutifs, administration relayée par une corticothérapie orale. Il apparaît donc clairement que la corticothérapie utilisée dans les formes plus sévères de LED n'est pas sans incidence sur la santé et la qualité de vie des patients.

Enfin, dans certains cas, des composés immunosuppresseurs sont utilisés. L'emploi des traitements immunosuppresseurs dans la maladie lupique ne se conçoit qu'avec discernement. En effet, leurs risques (infections à court terme, stérilité, oncogenèse possible à long terme) en font limiter l'indication aux formes viscérales graves ou cortico-dépendantes. Divers agents sont utilisés dans les schémas traditionnels: cyclophosphamide (Endoxan® par exemple) à la dose de 2 à 3 mg/kg/jour, azathioprine (Imurel® par exemple) à la dose de 2 à 4 mg/kg/jour, pour une durée de 6 mois à 2 ans. Outre leurs risques communs, le cyclophosphamide expose plus particulièrement aux cystopathies et aux cancers vésicaux. L'administration intraveineuse discontinue de cyclophosphamide (0,5 à 1 g/m² tous les mois pendant 6 mois puis tous les trimestres pendant 2 ans) associée à une corticothérapie à doses modérées est plus efficace que la seule corticothérapie. Elle est largement employée depuis quelques années.

Il apparaît clairement de la description ci-dessus que les traitements actuels sont purement symptomatiques et induisent de multiples effets secondaires affectant significativement la qualité de vie des patients. Aucun traitement satisfaisant du LED n'est donc disponible à ce jour. Il est donc nécessaire de trouver de nouveaux traitements capables de retarder la survenue des manifestations les plus graves, notamment la néphropathie lupique, de diminuer leur sévérité une fois qu'elles sont apparues, et surtout n'induisant pas d'effets secondaires aussi importants que les traitements actuels.

Les phosphodiestérases (PDE) forment une superfamille comprenant 11 familles d'enzymes responsables de l'hydrolyse de l'AMPc et du GMPc, seconds messagers intracellulaires, jouant ainsi un rôle majeur dans le contrôle normal et pathologique de la réponse cellulaire. Leur rôle essentiel dans la signalisation intracellulaire a fait de ces enzymes de nouvelles cibles thérapeutiques. Dans l'enthousiasme généré par la découverte de ces nouvelles cibles et de leur distribution tissulaire variée, on a assisté à l'émergence d'une littérature fournie sur les applications thérapeutiques potentielles des inhibiteurs de PDE. En particulier, de très nombreuses demandes de brevet ont été déposées concernant les applications thérapeutiques d'inhibiteurs des différentes familles de PDE. Dans à peu près toutes ces demandes, les applications thérapeutiques envisagées pour les inhibiteurs d'une famille particulière de PDE sont extrêmement larges, couvrant de nombreuses classes de maladies, aussi bien vasculaires que neurologiques, hématologiques ou inflammatoires, le nombre de maladies citées dans chaque catégorie étant très élevé. Notamment, au vu de ces demandes, il pourrait à priori sembler que le LED puisse être traité indifféremment par des inhibiteurs de l'une quelconque des familles PDE. En effet, le Tableau 1 ci-dessous regroupe un certain nombre de références de demandes de brevet concernant la possibilité d'utiliser des inhibiteurs des différentes familles PDE pour le traitement du lupus.

**Tableau 1. Références de demandes de brevet concernant la possibilité d'utiliser des inhibiteurs des différentes familles PDE pour le traitement du lupus**

| **Famille PDE** | **DEPOSANT** | **N° publication** | **TITRE** |
|---|---|---|---|
| 1 | BAYER HEALTHCARE | WO04081563 | DIAGNOSTICS AND THERAPEUTICS FOR DISEASES ASSOCIATED WITH PHOSPHODIESTERASE 1A (PDE1A) |
| 2 | CELL PATHWAYS, INC. | WO03017926 | TREATMENT FOR LUPUS ERYTHEMATOSUS |
| | BEAVO, JOSEPH, A. | WO0222661 | NOVEL PDES AND USES THEREOF |
| 3 | BAYER HEALTHCARE | WO04071377 | DIAGNOSTICS AND THERAPEUTICS FOR DISEASES ASSOCIATED WITH PHOSPHODIESTERASE 3A (PDE3A) |
| 4 | OHSHIMA ETSUO | US 6,716,987 | DERIVATIVES OF BENZOFURAN OR BENZODIOXAZOLE COMPOUNDS |
| | PITTS WILLIAM J ET AL. | US2003/04974 | DUAL INHIBITORS OF PDE7 AND PDE4 |
| 5 | WOOD, RALPH, E., | WO0202118 | METHOD OF TREATING PERIPHERAL VASCULAR DISEASES, PERIPHERAL NEUROPATHIES, AND AUTONOMIC NEUROPATHIES |
| 6 | AMBIT BIOSCIENCES CORPORATION | WO04110998 | PYRROLE COMPOUNDS AND USES THEREOF |
| 7 | DAIICHI SUNTORY PHARMA CO.,LTD | WO04111053 | IMIDAZOTRIAZINONE DERIVATIVES AS PDE 7 (PHOSPHODIESTERASE 7) INHIBITORS |
| 8 | INCYTE GENOMICS, INC | US6080548 | CYCLIC NUCLEOTIDE PHOSPHODIESTERASES |
| 9 | INCYTE GENOMICS, INC | US5922595 | CYCLIC GMP PHOSPHODIESTERASE |
| 10 | INCYTE GENOMICS, INC | US6416991 | HUMAN CYCLIC NUCLEOTIDE PDES |
| 11 | BAYER HEALTHCARE | WO04029617 | DIAGNOSTICS AND THERAPEUTICS FOR DISEASES ASSOCIATED WITH PHOSPHODIESTERASE 11A (PDE11A) |

Il n'est bien entendu pas possible que les inhibiteurs de toutes les familles de PDE soient réellement efficaces pour le traitement de la maladie très particulière et surtout très polymorphe qu'est le LED. De plus, à l'exception de la demande W003017926 qui suggère l'utilisation d'inhibiteurs de PDE2, et éventuellement de PDE5, pour le traitement spécifique du LED, aucune des demandes citées dans le Tableau 1 ci-dessus ne présente aucun résultat susceptible d'inciter un homme du métier à penser que les inhibiteurs des autres familles de PDE pourraient réellement avoir un effet quelconque dans le traitement du lupus.

Au vu de la littérature extrêmement fournie et discordante concernant les inhibiteurs de PDE et leurs applications thérapeutiques potentielles, il est clair qu'un homme du métier n'aurait pas été incité à chercher un nouveau composé efficace pour le traitement du lupus parmi les inhibiteurs de PDE, à l'exception peut-être des inhibiteurs de PDE2.

En outre, concernant plus précisément les inhibiteurs de PDE4, il est important de souligner qu'aucun des documents de l'art antérieur ne décrit de résultats expérimentaux concernant le LED, mais seulement des résultats montrant la capacité des inhibiteurs particuliers décrits dans ces documents à inhiber des phénomènes allergiques et inflammatoires, voire dans certains cas uniquement leur capacité à inhiber la production de TNF-α induite par le LPS.

Ainsi, le brevet US 6,716,987 montre dans la partie expérimentale que les composés inhibiteurs de PDE4 revendiqués ont un effet inhibiteur sur plusieurs phénomènes allergiques et inflammatoires complètement différents du LED, et en particulier sur la production de TNF-α induite par le LPS. Aucun résultat visant le LED n'est décrit.

De même, dans la demande US2003/0104974, seule la capacité des composés ayant un double effet inhibiteur sur PDE4 et PDE7 revendiqués à inhiber la sécrétion de TNF-α induite par le LPS est exemplifiée.

WO 2004/014913 décrit l'utilisation d'une vaste famille de dérivés de l'adénine en tant qu'inhibiteurs de la PDE4 pour le traitement d'un grand nombre de maladies inflammatoires, parmi lesquelles aussi le LES.

Ainsi, les documents de l'art antérieur concernant des inhibiteurs de PDE4 et revendiquant ou suggérant de façon générale leur utilisation dans le traitement du LED ne démontrent expérimentalement qu'une action anti-inflammatoire ou anti-allergique des composés revendiqués. Aucun effet sur des composantes pourtant essentielles de la maladie lupique telles que la protéinurie ou la présence d'anticorps anti-ADN sérique, n'est décrit.

L'homme de l'art travaillant dans le domaine du traitement du LED, conscient de la complexité et de l'aspect polymorphe de.la maladie lupique, n'aurait certainement pas considéré les résultats présentés dans les documents de l'art antérieur comme suffisants pour l'inciter à tester les inhibiteurs de PDE4 dans le traitement du lupus.

La demande EP 1 043 021 décrit des composés dérivés de l'adénine et leur utilisation en tant qu'inhibiteur sélectif de la réponse immunitaire des cellules TH2 ou en tant qu'agent anti-allergique.

Bien que les applications thérapeutiques décrites dans cette demande visent principalement les maladies allergiques induites par une réponse TH2, le lupus est également mentionné.

Toutefois, les exemples expérimentaux montrent seulement que les composés revendiqués ont un effet inhibiteur sur la production de cytokines par des cellules TH2 sensibilisées, sur l'infiltration des éosinophiles, ainsi qu'un effet anti-allergique.

Aucun résultat expérimental montrant un effet quelconque de ces composés dans un mécanisme particulièrement important dans le LED n'est donc décrit, et la simple allusion au lupus dans la description n'aurait donc certainement pas inciter l'homme de l'art travaillant dans le domaine du traitement du lupus à tester ces composés dans le traitement du LED.

De façon surprenante, les inventeurs ont pourtant pu mettre en évidence l'efficacité dans le traitement du LED de composés dérivés de l'adénine, substitués en positions 2 et 9, et éventuellement N(6) de l'adénine, et possédant des capacités inhibitrices des enzymes de la famille PDE4. Les inventeurs ont en effet démontré que ces composés sont capables d'inhiber la progression de la maladie chez des souris MRL/lpr, modèle murin du LED, notamment en s'opposant à la néphropathie (diminution de la protéinurie), à la production de TNF-α, et au développement d'auto-Ac anti-ADN, marqueurs de la maladie, conduisant ainsi à une survie prolongée des souris MRL/lpr.

En outre, les inventeurs ont comparé l'effet de ces composés par rapport à un autre inhibiteur de PDE4 dérivé de l'adénine, la denbufylline, sur les différentes composantes de la maladie lupique chez des souris MRL/lpr. La formule chimique de la denbufylline est présentée ci-dessous :

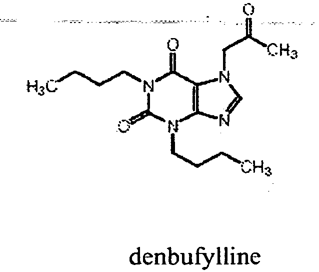

Les résultats obtenus montrent que les composés selon l'invention possèdent un effet bien supérieur à la denbufylline pour le traitement du lupus in vivo. En effet, bien que la denbufylline ait un effet similaire sur l'inhibition de la production de TNF-α, les effets de ce composé sur la protéinurie ou le développement d'auto-anticorps anti-ADN, deux composantes essentielles de la maladie lupique sont nettement inférieurs à ceux des composés selon l'invention voire inexistants.

Ainsi, les inventeurs ont mis en évidence une classe de composés dérivés de l'adénine, substitués en positions 2 et 9, et éventuellement N(6) de l'adénine, et démontrent pour la première fois un véritable effet in vivo sur toutes les composantes de la maladie très polymorphe qu'est le LED.

L'invention concerne donc l'utilisation d'un composé de formule (I) 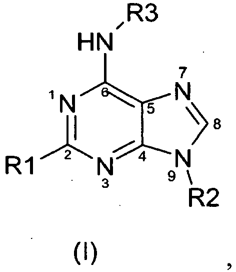 où
R1 est choisi parmi CF₃, un alkyle en C₁-C₅, ou (CH₂)ₙR4 où n est de 0 à 4;
R2 est choisi parmi (CH₂)ₘR4 ou (CH₂)ₘAr où m est de 0 à 5;
R3 est choisi parmi un hydrogène ou un méthyle ;
R4 est choisi parmi un phényle, OH, un alcoxy en C₁-C₃, un dialkylamino en C₁-C₃, pipéridino, ou N-méthylpipérazino ;
Ar représente 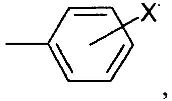 où X est choisi parmi F, Cl, , un alcoxy en C₁-C₃, ou CF₃ ;
ou d'un sel pharmaceutiquement acceptable, d'un énantiomère ou d'un diastéréoisomère de celui-ci, ou d'un mélange de ceux-ci pour la fabrication d'un médicament destiné au traitement du lupus érythémateux disséminé.

Par « alkyle en C₁-Cᵢ », i ≥ 1, on entend dans la présente demande un radical hydrocarboné saturé linéaire ou ramifié de formule -CⱼH₂ⱼ₊₁, où 1 ≤ j ≤ i. Notamment, un alkyle en C₁-C₅ peut être un alkyle en C₁ (méthyle), C₂ (éthyle), C₃ (n-propyle, ou isopropyle), C₄ (n-butyle, isobutyle, sec-butyle, ou tert-butyle), ou C₅ (ex: n-pentyle, néopentyle, isopentyle, tert-pentyle). De même, un alkyl en C₁-C₃ peut être un alkyle en C₁ (méthyle), C₂ (éthyle), ou C₃ (n-propyle, ou isopropyle).

Par « alcoxy en C₁-C₃», on entend un radical de formule -O(alkyle en C₁-C₃), où l'alkyle en C₁-C₃ est tel que définit précédemment. Ainsi, le terme comprend les radicaux méthoxy, éthoxy, n-propyloxy et isopropyloxy. Des alcoxy avantageux dans l'invention sont le méthoxy et le n-propyloxy, en particulier le méthoxy.

Par « dialkylamino en C₁-C₃», on entend un radical de formule -N(alkyle en C₁-C₃)₂, où chaque alkyle en C₁-C₃ est indépendamment tel que définit précédemment. Avantageusement, les deux alkyles en C₁-C₃ sont identiques. Des dialkylamino en C₁-C₃ avantageux dans l'invention possèdent une formule choisie parmi -N(CH₃)₂, - N(C₂H₅)₂, ou -N(C₃H₇)₂.

Les énantiomères et les diastéréoisomères sont des stéréoisomères. Par « stéréoisomères », on entend des isomères, c'est-à-dire des composés de même formule brute, ayant la même formule développée mais une disposition spatiale différente. Des « énantiomères » sont alors des composés stéréoisomères images l'un de l'autre dans un miroir plan mais non superposables, tandis que des « diastéréoisomères » sont des stéréosiomères qui ne sont pas des énantiomères, c'est-à-dire qui ne sont pas stéréoisomères images l'un de l'autre dans un miroir plan. La portée de l'invention s'étend aux différents énantiomères et diastéréoisomères de formule (I), ainsi qu'à leurs mélanges, notamment à un mélange racémique de stéréoisomères.

La présence d'un groupe méthyle en position N(6) de l'adénine améliore l'efficacité des composés définis précédemment pour le traitement du lupus. Dans un mode de réalisation avantageux, R3 est donc un méthyle.

La présence en position 9 de l'adénine d'un substituant benzyle, éventuellement substitué, améliore l'efficacité des composés définis précédemment pour le traitement du lupus. Dans un mode de réalisation avantageux, R2 représente donc (CH₂)Ar, où Ar est tel que défini précédemment. Le substituant X du groupe Ar peut se trouver en toute position du cycle benzénique. Toutefois, le substituant X du groupe Ar se trouve avantageusement en position 2 du cycle benzénique. En outre, X est avantageusement choisi parmi un atome de fluor et un méthoxy.

Des composés particuliers susceptibles d'être utilisés pour la fabrication d'un médicament destiné au traitement du LED ont été identifiés par les inventeurs. Notamment, de tels composés avantageux comprennent les composés représentés dans le Tableau 2 suivant.

**Tableau 2. Composés avantageux**

| Nom composé | Formule semi-développée* | Nom nomenclature |
|---|---|---|
| NCS 613 | 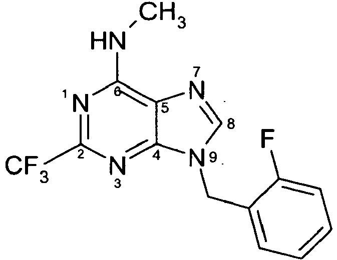 | 9-(2-fluorobenzyl)-N(6)-méthyl-2-trifluorométhyladénine |
| NCS 700 | 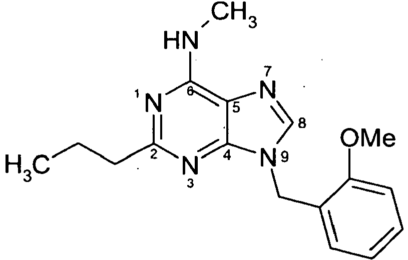 | 9-(2-méthoxybenzyl)-N(6)-méthyl-2-n-propyladénine |
| NCS 728 | 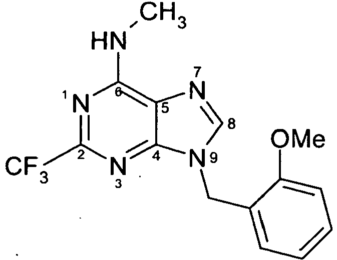 | 9-(2-méthoxybenzyl)-N(6)-méthyl-2-trifluorométhyladénine |
| NCS 658 | 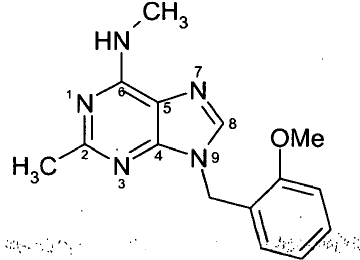 | 9-(2-méthoxybenzyl)-N(6)-méthyl-2-méthyladénine |

| | | |
|---|---|---|
| *Me = CH₃ | | |

Un composé particulièrement avantageux est la 9-(2-fluorobenzyl)-N(6)-méthyl-2-trifluorométhyladénine (NCS 613).

Deux voies de synthèse peuvent être utilisées pour la préparation des composés utilisés selon l'invention pour la fabrication d'un médicament destiné au traitement du LED.

La première est classique, peu convergente et utilise la chloropurine correctement substituée en position R₂, qui est d'abord alkylée avec différents halogénures d'alkyle (R₂X), puis aminée par réaction avec la N-méthylamine (voir Schéma 1 ci-dessous). Davantage de détails concernant ce procédé de préparation peuvent être trouvés dans Bourguignon, J.J et al., J. Med. Chem. (1997) 40, 1768-1770.

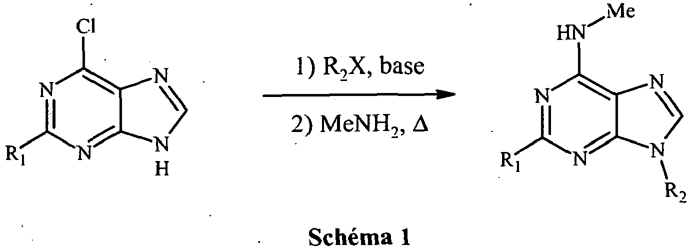

La seconde voie de synthèse permet une exploration plus systématique de la position 2 de l'adénine par réaction d'amination, ou de couplage au palladium (0) (Suzuki, Sonogashira, Heck, Buchwald) à partir du dérivé 2-iodé correspondant. Ce dernier est obtenu par traitement à l'isoamyl nitrite puis à l'aide de diiodométhane du dérivé 2-amino (voir schéma 2 ci-dessous). Davantage de détails concernant cette deuxième voie de synthèse peuvent être trouvés dans Raboisson P. et al., Eur. J. Med. Chem. (2003) 38, 199-214).

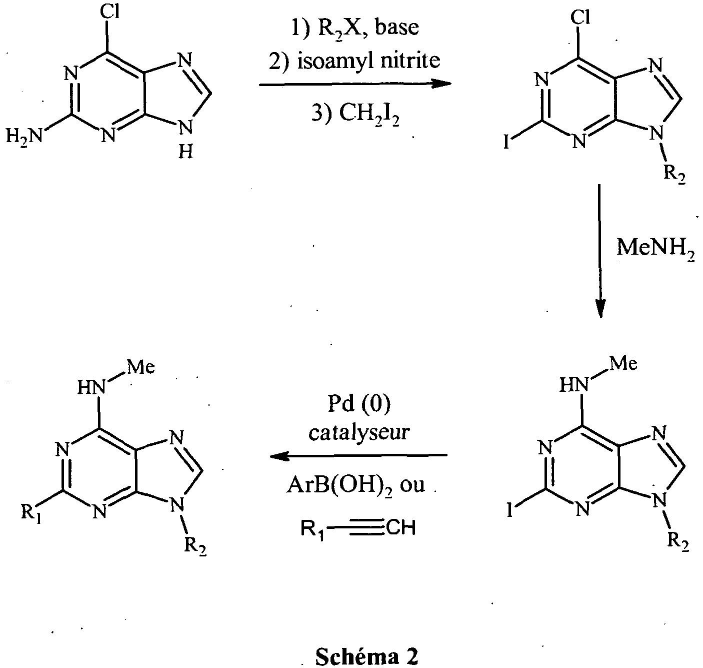

Les composés décrits précédemment peuvent être utilisés directement ou en tant que sels pharmaceutiquement acceptables. Par « sel pharmaceutiquement acceptable » on entend notamment des sels d'addition d'acides inorganiques tels que chlorhydrate, sulfate, phosphate, diphosphate, bromhydrate et nitrate ou d'acides organiques tels que acétate, maléate, fumarate, tartrate, succinate, citrate, lactate, méthane sulfonate, p-toluènesulfonate, pamoate, oxalate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical salts", J Pharm. Sci. 66:1 (1977).

Un médicament destiné au traitement du LED fabriqué à partir d'un composé décrit précédemment peut en outre comprendre un support pharmaceutiquement acceptable connu de l'homme du métier. Un tel médicament peut également contenir tout type d'excipient pharmaceutiquement acceptable. Ces excipients peuvent notamment servir à améliorer la conservation des composés contenus dans le médicament, leur biodisponibilité, ou à permettre une libération prolongée des principes actifs dans l'organisme.

Un médicament destiné au traitement du LED fabriqué à partir d'un composé décrit précédemment peut être employé par différentes voies d'administration, notamment par voie orale, nasale, rectale, intraveineuse, intramusculaire, sous-cutanée, topique. En particulier, le LED est caractérisé à la fois par des manifestations systémiques telles que l'inflammation générale, ou la circulation de complexes immuns, et par des manifestations locales, notamment cutanées ou articulaires. Les médicaments selon l'invention peuvent donc être utilisés par voie invasive ou non invasive, par exemple intraveineuse, intramusculaire, sous-cutanée, orale, nasale, topique ou par infiltration au niveau des articulations.

Les inventeurs ont démontré que les composés décrits précédemment sont particulièrement efficaces pour le traitement du LED. Ces composés peuvent donc être utilisés seuls. Ils peuvent aussi être utilisés en association avec un deuxième composé utile pour le traitement du lupus. De tels composés utiles pour le traitement du lupus sont de préférence choisis parmi les anti-inflammatoires non stéroïdiens, les anti-paludéens de synthèse, les corticoïdes ou les immunosuppresseurs. Ainsi, dans un mode de réalisation, l'invention concerne l'utilisation d'un composé tel que défini précédemment, en association avec un deuxième composé choisi parmi les anti-inflammatoires non stéroïdiens, les anti-paludéens de synthèse, les corticoïdes ou les immunosuppresseurs, pour la fabrication d'un médicament destiné au traitement du lupus érythémateux disséminé, pour une administration simultanée, séparée, étalée dans le temps ou alternée.

Les anti-inflammatoires non stéroïdiens (AINS) sont des médicaments symptomatiques à action rapide qui ont des propriétés analgésique, antipyrétique et anti-inflammatoire et qui, malgré leur hétérogénéité chimique, ont un mode d'action commun: diminution de la production tissulaire des prostaglandines (PG) et thromboxanes (TX), par inhibition de la cyclooxygénase (COX) dont il existe deux iso-enzymes (COX-1, constitutive et ubiquitaire et COX-2, inductible dans les monocytes macrophages et les polynucléaires). Au sens de l'invention, les AINS regroupent les AINS proprement dit et les salicylés, car ils possèdent à peu près tous les mêmes effets thérapeutiques et les mêmes effets indésirables. Les AINS utiles en association avec un composé tel que décrit précédemment dans un médicament selon l'invention comprennent les AINS salicylés tels que le diflunisal, le benorilate ou l'aspirine ; les dérivés propioniques tels que l'alminoprofène, le kétoprofène, l'ibuprofène, le naproxène, le flurbiprofène ou l'acide tiaprofénique ; les dérivés indoliques tels que l'indométacine, le sulindac ou l'étodolac ; les dérivés pyrazolés tels que la phénylbutazone ; les oxicams tels que le piroxicam, le ténoxicam, ou le méloxicam ; les anti-COX2 sélectifs tels que le rofecoxib ou le celecoxib ; ou encore d'autres AINS tels que le diclofénac, le nimésulide, l'acide niflumique, l'acide méfénamique, ou la nabumétone. De préférence, l'AINS utilisé en association avec un composé précédemment décrit est choisi parmi l'aspirine, l'indométacine et l'ibuprofène.

Les anti-paludéens de synthèse susceptibles d'être associés à un composé dérivé de la 9-benzyladénine décrit précédemment dans un médicament selon l'invention comprennent les 4-méthanolquinoléines telles que la quinine et la méfloquine ; les 4-aminoquinoléines telles que la chloroquine, l'hydroxychloroquine et l'amodiaquine ; les 8-aminoquinoléines telles que la primaquine ; les biguanides tels que le proguanil ; les sulfonamides associées aux diaminopyrimidines telles que la sulfadoxine/pyriméthamine ; les sesquiterpènes lactones telles que l'artémisinine et dérivés. De préférence, l'anti-paludéen de synthèse utilisé en association avec un composé précédemment décrit est choisi parmi la chloroquine et hydroxychloroquine, de préférence l'hydroxychloroquine.

Les corticoïdes sont formés des corticoïdes naturels (cortisone et hydrocortisone) et de leurs dérivés de synthèse. Les corticoïdes utiles en association avec un composé dérivé de la 9-benzyladénine décrit précédemment dans un médicament selon l'invention comprennent la cortisone, l'hydrocortisone, la prednisone, la prednisolone, la méthylprednisolone, la triamcinolone fluorée, la dexaméthasone, la bétàméthasone, la paraméthasone, la triamcinolone, le cortivazol, et le tétracosactide. La prednisone reste le corticoïde de référence dans le traitement du lupus et est utilisée préférentiellement. En cas d'association d'un corticoïde avec un composé tel que décrit précédemment dans un médicament selon l'invention, un régime sans sel et de type diabétique (réduit en glucides et en calories) et des supplémentations en potassium, calcium, vitamine D sont indispensables.

Enfin, les immunosuppresseurs susceptibles d'être associés à un composé dérivé de la 9-benzyladénine décrit précédemment dans un médicament selon l'invention comprennent la cyclosporine, le tacrolimus, l'azathioprine et le cyclophosphamide. De préférence, l'immunosuppresseur utilisé en association avec un composé précédemment décrit est choisi parmi l'azathioprine et le cyclophosphamide.

Lorsqu'un médicament destiné au traitement du LED est préparé à partir d'un composé tel que décrit précédemment et d'un deuxième composé utile dans le traitement du LED, ces composés actifs peuvent être administrés de façon simultanée, séparée, étalée dans le temps ou alternée. En effet, le médicament résultant de l'association des deux composés peut être présenté sous la forme d'un médicament unique, chaque prise entraînant nécessairement l'administration des deux composés de façon « simultanée », ou bien sous la forme de deux unités de médicament distinctes comprenant chacune l'un des composés, l'administration pouvant alors être faite de façon « séparée » (en même temps, mais dans deux unités distinctes, éventuellement par des voies différentes) ou bien « étalée dans le temps » (les deux unités étant administrées à des moments différents, à quelques heures ou quelques jours d'écart). Ces différentes possibilités permettent de tenir compte des propriétés particulières des différents composés. En outre, les deux composés peuvent être administrés de façon « alternée », c'est-à-dire que le patient est traité d'abord par une cure de l'un des composés, puis par une cure du deuxième composé, et ainsi de suite de façon alternée.

### DESCRIPTION DES FIGURES

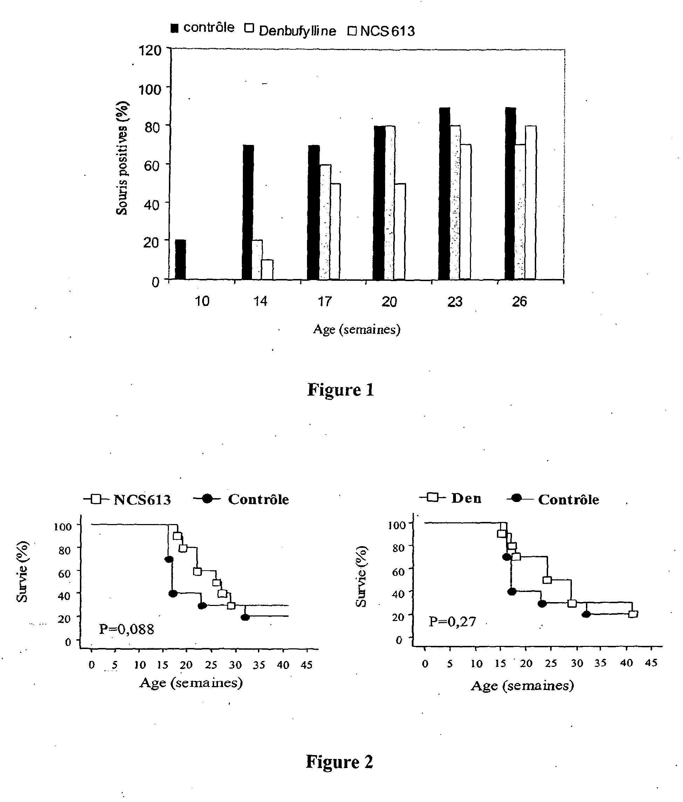

**Figure 1****: Effet du composé NCS 613 et de la denbufylline sur la protéinurie chez les souris MRL/lpr.** Les souris ont reçu jusqu'à l'âge de 13 semaines 4 administrations soit de 30 µg de composé NCS 613 soit de 100 µg de denbufylline. Les résultats représentent le pourcentage de souris ayant une protéinurie positive en fonction de l'âge (en semaines) des souris utilisées.
**Figure 2****: Effet du composé NCS 613 (A) et de la denbufylline (B) sur la survie des souris lupiques MRL/lpr.** Les souris ont reçu jusqu'à l'âge de 13 semaines 4 administrations soit de 30 µg de composé NCS 613 soit de 100 µg de denbufylline. Les résultats sont exprimés en pourcentage de survie des souris. Les souris contrôles n'ont reçu que du PBS (solution saline tamponnée par du phosphate, pH 7.4) sans le principe actif.

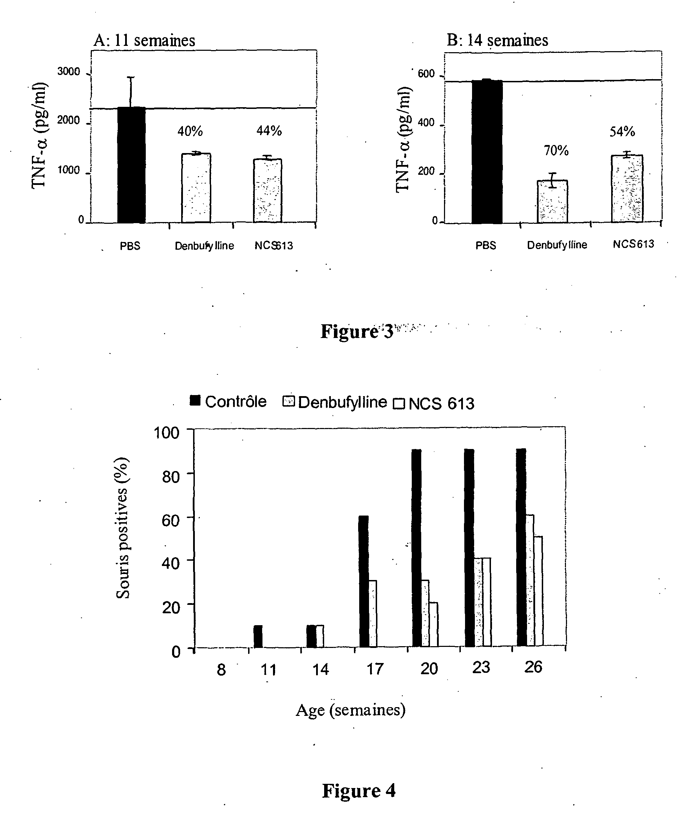

**Figure 3****: Effet du composé NCS 613 et de la denbufylline sur la production de TNF-α par les cellules issues du sang des souris traitées après stimulation par du LPS.** La sécrétion du TNF-α par les cellules issues du sang des souris ayant reçu la solution saline (PBS), le composé NCS 613 ou la denbufylline après stimulation ex vivo par du LPS pendant 24h a été mesurée dans les surnageants par ELISA. Les barres d'erreur représentent la déviation standard des duplicata, et le pourcentage d'inhibition de la sécrétion de TNF-α en fonction de la sécrétion mesurée dans le groupe PBS est indiqué. Les figures représentent des expériences réalisées sur des souris âgées de 11 (A) et 14 (B) semaines.
**Figure 4****: Effet du composé NCS 613 et de la denbufylline sur la production d'Ac anti-ADN double brin.** La présence d'Ac dirigés contre l'ADN dans les sérums de souris a été évaluée à différents âges (en semaines) par test ELISA. Les résultats sont représentés en pourcentage de souris positives. Une souris est définie comme positive lorsque la densité optique (DO) mesurée en ELISA est supérieure à 1.

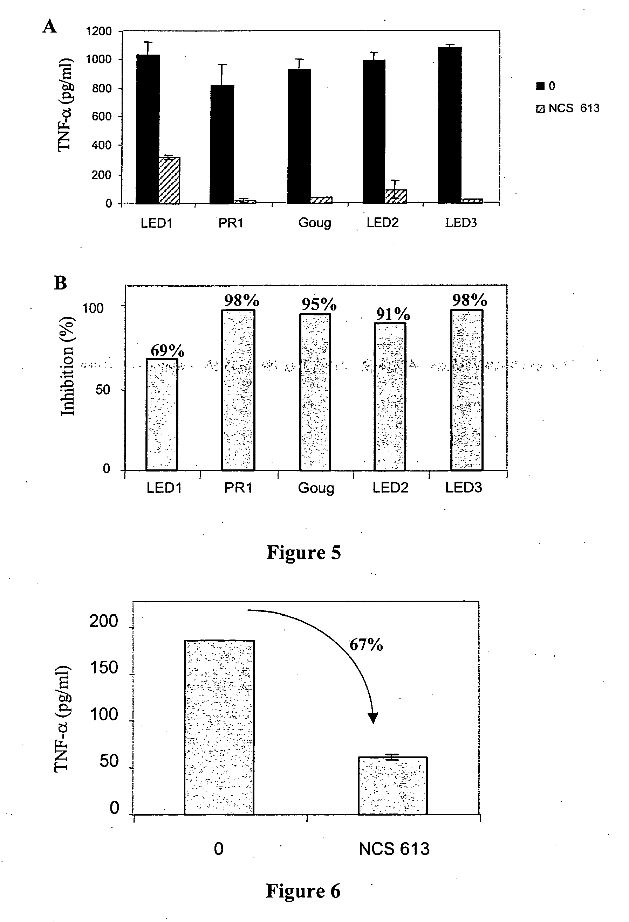

**Figure 5****. Inhibition de la sécrétion de TNF-α par le composé NCS 613 après stimulation des PBMCs de patients atteints de maladies auto-immunes par du LPS.** Les cellules issues du sang de patients atteints de lupus (LED), de polyarthrite rhumatoïde (PR) et d'un syndrome de Gougerot-Sjôgren (Goug) sont incubées pendant 45 minutes en présence ou non de NCS 613 (10µM), puis stimulées par du LPS. Les surnageants sont prélevés après 24h de culture et le dosage du TNF-α dans les surnageants est réalisé par ELISA. Les résultats sont représentés en concentration de TNF-α après incubation en présence ou en absence de NCS 613 (A) ou en inhibition de la sécrétion de TNF-α après incubation avec le NCS 613 (B). Les barres d'erreur représentent la déviation standard des duplicata.
**Figure 6****. Inhibition de la sécrétion de TNF-α par les PBMCs d'un patient lupique après incubation avec le NCS 613.** Les cellules issues du sang d'un patient atteint de lupus (LED2) sont incubées en présence ou non de NCS 613 (10µM). Les surnageants sont prélevés après 24h de culture et le dosage du TNF-α dans les surnageants est réalisé par ELISA. Les résultats sont représentés en concentration de TNF-α après incubation en présence ou en absence de NCS 613 et le pourcentage d'inhibition de la sécrétion de TNF-α après incubation avec le NCS 613 est indiqué. Les barres d'erreur représentent la déviation standard des duplicata.

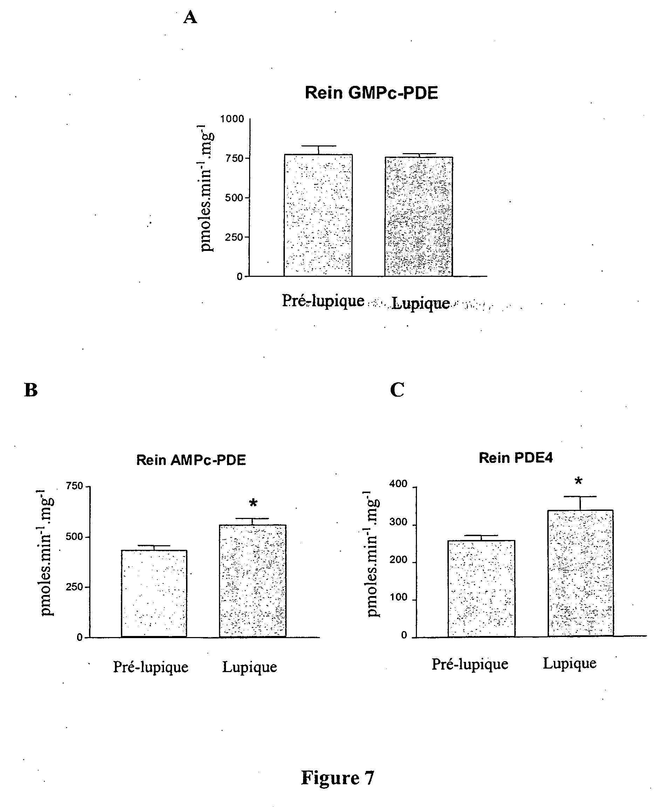

**Figure 7****. Variation des activités phosphodiestérasiques au niveau du tissu rénal au cours de l'installation du lupus.** L'activité d'hydrolyse du GMPc (A), de l'AMPc (B) ainsi que l'activité PDE4 (C) ont été comparées chez des souris pré-lupiques (8 semaines, n=3) et lupiques (18 semaines, n=3). Les résultats représentent les moyennes des résultats obtenus sur les trois souris ±s.e.m. exprimés en pmol.min⁻¹.mg⁻¹.*P<0,05 en comparaison avec les tissus pré-lupiques.

### EXEMPLES

### EXEMPLE 1 : Effets biologiques du composé NCS 613 et comparaison avec les effets d'un autre inhibiteur de PDE4, la denbufylline

### 1.1 Matériels et méthodes

### 1.1.1 Expérimentation animale : étude in vivo de l'effet de l'administration du composé NCS 613 ou de la denbufylline

### Administration et prélèvements

Des souris MRL/lpr pré-autoimmunes femelles âgées de 5 semaines, ont reçu par voie intraveineuse, soit la solution saline tamponnée (PBS ; groupe contrôle, 100µl par souris), soit le composé NCS 613 (30µg/100µl par souris, dilué en solution saline contenant 10% d'éthanol), soit la denbufylline (100 µg/100 µl par souris, dilué en solution saline contenant 10% d'éthanol). Les deux groupes de souris sont composés chacun de 10 animaux. Les administrations (au nombre de 4) ont été effectuées aux âges de 5, 7, 9 et 13 semaines, et les animaux ont été suivis régulièrement (observations, dosage de la protéinurie sur bandelette Albutix, prélèvements sanguins).

### Dosage des anticorps anti-ADN dans le sérum.

La présence d'Ac dirigés contre l'ADN dans les sérums de souris a été évaluée à différents âges par test ELISA (Monneaux et al., Eur. J. Immunol. (2003) 33, 287-296 ; Monneaux et al., Arthritis Rheum (2004) 50, 3232-3238.). L'ADN double brin de thymus de veau (Sigma) a été adsorbé (100ng/ml) sur des plaques 96 puits en chlorure de polyvinyle (Falcon) et incubé une nuit à 37°C. Après trois lavages et une étape de saturation en tampon PBS-Tween contenant de l'albumine sérique bovine, les sérums sont incubés (dilués au 1/500) pendant une heure à 37°C. La révélation de la liaison des Ac présents dans le sérum à l'ADN est réalisée par une incubation avec un Ac secondaire (anti-IgG de souris couplé à la peroxydase), suivie d'une incubation avec le substrat de la peroxydase et d'un chromogène. L'absorbance est mesurée à 450 nm au spectrophotomètre après arrêt de la réaction.

### Dosage de la sécrétion de TNF-α par les cellules issues du sang des souris traitées après stimulation par du LPS.

La sécrétion du TNF-α a été mesurée après stimulation des cellules issues du sang de souris (pool du sang des 7 souris par groupe) par des lipopolysaccharides bactériens (LPS). La stimulation des cellules par du LPS pendant 24h permet de générer la sécrétion de TNF-α qui peut ensuite être mesurée dans les surnageants de culture. Les cellules issues du sang des 3 groupes de souris ont été purifiées par gradient de Ficoll, et ensuite stimulées par du LPS. Les surnageants ont été récupérés après 24h de culture, et la présence de TNF-α dans les surnageants a été mesurée par un test ELISA double sandwich (BD Biosciences). Cette expérience a été réalisée à différents âges (11 et 14 semaines, soit 2 semaines après la 3^{ème} administration et 1 semaine après la 4^{ème} administration respectivement).

### 1.1.2 Etude chez les patients : étude ex vivo de l'effet du composé NCS 613 sur la production de TNF-α

Le sang de 5 patients atteints de lupus, polyarthrite rhumatoïde ou syndrome de Gougerot-Sjögren est prélevé directement sur des tubes héparinés. Les cellules mononucléées du sang sont purifiées par centrifugation (30 min à 600g) sur un gradient de Ficoll. Les cellules sont ensuite mises en culture à raison de 2x10⁵ cellules par puits en présence ou non du composé NCS 613 (10µM) pendant 45 minutes puis stimulées par du LPS. Les surnageants sont prélevés après 24h de culture et le dosage du TNF-α dans les surnageants est réalisé par test ELISA double sandwich (BD Biosciences).

### 1.1.3 Etude sur les phosphodiestérases des nucléotides cycliques

### Activités phosphodiestérasiques

L'activité de la phosphodiestérase des nucléotides cycliques a été déterminée à l'aide d'une méthode radioenzymatique en utilisant de l'AMP ou du GMP cyclique tritié (1µM) comme substrat (Lugnier C. et al., Biochem. Pharmacol. (1986) 35, 1743-1751.; Keravis T et al., Meth. Mol. Biol. (2005) 307, 63-74.). L'adénosine ou le guanosine monophosphate tritié formé par hydrolyse du nucléotide cyclique marqué a été, dans une seconde incubation avec une nucléotidase en excès, transformé en adénosine ou guanosine tritié. Le nucléoside formé a été séparé des nucléotides par chromatographie sur une résine échangeuse d'anions. La radioactivité du nucléoside a été déterminée par scintillation liquide. Les incubations enzymatiques ont été effectuées dans les conditions où il n'y a pas plus de 15% d'hydrolyse du substrat, chaque point étant répété en double.

### Isolement des PDE1, PDE3, PDE4 et PDE5

Un segment de 3 g de média d'aorte bovine fragmenté à l'aide de ciseaux a été homogénéisé à l'aide d'un ultra-turrax puis d'un potter verre-verre dans 7 volumes/poids de tampon A contenant un cocktail d'inhibiteurs de protéases (20 mM Tris-HCl, 0,25 M saccharose, 2 mM acétate de Mg, 1 mM dithiothreitol, 5 mM EGTA, 2000 U/ml aprotinine, 10 mg/l leupeptine et 10 mg/l d'inhibiteur trypsique de soja). L'homogénat a été centrifugé pendant 1 h à 105,000 g. Le surnageant a été déposé sur une colonne de DEAE-Sephacel (15 X 1,6 cm), pré- équilibrée avec le tampon B (tampon A dépourvu de saccharose, d'EGTA et d'inhibiteurs de protéases). La colonne a été lavée jusqu'à ce qu'aucune absorption ne puisse être détectée à 280 nm, puis éluée avec un gradient linéaire en NaCl (0-0,5M) dans le tampon B. Des fractions de 3 ml ont été recueillies et les activités enzymatiques ont été déterminées dans diverses conditions de façon à caractériser les pics d'activité enzymatique contenant suivant l'ordre d'élution la PDE1, la PDE5, la PDE3 et la PDE4. Chaque fraction aliquotée est conservée à -80°C.

### Isolement de la PDE2

Les plaquettes humaines lavées fournies par l'Etablissement Français du Sang de Strasbourg ont été culottées, homogénéisées dans le tampon A et centrifugées pendant 1 h à 105,000 g. Le surnageant a été soumis à chromatographie suivant la procédure décrite pour la média d'aorte (C-2). Puis les fractions riches en PDE2 (activité stimulée par le GMPc 5µM) ont été soumises à chromatographie HPLC MonoQ H/R 5X 5 Pharmacia. La fraction contenant la PDE2 est aliquotée et conservée à -80°C (Kameni Tcheudji J.F et al., J. Mol. Biol. (2001) 310, 181-791.).

### Activité et profil pharmacologique des molécules

### Détermination de la CI₅₀

La concentration de substance qui inhibe de 50% l'activité enzymatique (CI₅₀) à 1µM de substrat a été calculée par régression non linéaire (Prism, GraphPad) à l'aide des résultats obtenus pour des concentrations des composés variant suivant la puissance de la molécule de 1nM à 300 µM. En raison de problèmes de solubilité, la concentration maximale utilisée dans les essais a été limitée à 300 µM.

La détermination des CI₅₀ sur l'ensemble des PDE1 à PDE5 permet de caractériser la spécificité d'une molécule pour sa cible.

### 1.2 Résultats

### 1.2.1 Capacité inhibitrice sélective de PDE4C du composé NCS 613

La sélectivité du composé NCS 613 et de la denbufylline vis-à-vis des différentes familles d'enzymes PDE a été analysée. Les résultats présentés dans le Tableau 3 ci-dessous montrent que ce composé est sélectif des enzymes de la famille PDE4.

**Tableau 3. Effets du composé NCS 613 sur les PDEs isolées de tissus vasculaires (CI₅₀, µM)**

| Substances | PDE1 | PDE2 | PDE3 | PDE4 | PDE5 |
|---|---|---|---|---|---|
| NCS 613 | 39 | 24 | n.s. | 0,042 | 4,7 |
| Denbufylline | 33 | 208 | n.s | 0,76 | 5,4 |

| | | | | | |
|---|---|---|---|---|---|
| n.s. : CI₅₀ > 300 µM. | | | | | |

De plus, la sélectivité du composé NCS 613 vis-à-vis des 4 sous-types d'enzymes PDE4 (A, B, C, D) a également été testée. Les résultats présentés dans le Tableau 4 ci-dessous indiquent que ce composé est sélectif du sous-type PDE4C.

**Tableau 4. Effets du composé NCS 613 sur les sous-types de PDE4 recombinantes (CI₅₀, nM)**

| Composé | PDE4A | PDE4B | PDE4C | PDE4D |
|---|---|---|---|---|
| NCS 613 | 43 | 50 | 1,4 | 14 |

### 1.2.2 Effet de l'administration du composé NCS 613 ou de la denbufylline à des souris pré-autoimmunes sur le développement de la maladie

L'étude *in vivo* des propriétés biologiques du composé NCS 613 a été réalisée chez des souris pré-autoimmunes MRL/lpr. Chez ces souris, la présence d'une mutation appelée lpr qui concerne le gène Fas (récepteur membranaire impliqué dans l'entrée des cellules en apoptose) conduit à une pathologie autoimmune, qui bien que semblable au lupus humain de par les signes cliniques et biologiques développés, est plus sévère que la pathologie observée chez l'homme. La lignée de souris MRL/lpr est caractérisée par un syndrome lympho-prolifératif associé à des lésions vasculaires, des arthrites et une glomérulonéphrite à l'origine de la mort de l'animal. L'apparition de la maladie chez les souris MRL/lpr est précoce (50% de mortalité à 20 semaines) et les premiers signes cliniques (protéinurie) apparaissent dès l'âge de 13-14 semaines. Comme dans le lupus humain, on retrouve chez les souris MRL/lpr la présence d'auto-Ac dirigés contre des complexes nucléaires (splicéosome et nucléosome). La lignée de souris MRL/lpr représente donc un très bon modèle murin du lupus érythémateux disséminé. Cependant, une autre caractéristique des souris MRL/lpr est le développement d'une arthrite accompagnée de facteurs rhumatoïdes semblable à une autre maladie auto-immune humaine, la polyarthrite rhumatoïde. En effet, vers l'âge de 3-4 mois, 45% des souris MRL/lpr présentent des infiltrations au niveau synovial accompagnées d'une érosion articulaire.

Les effets in vivo du composé NCS 613 sur les différentes composantes de la maladie lupique développée par les souris MRL/lpr, ont en outre été comparés aux effets d'un autre inhibiteur de PDE4 dérivé de l'adénine, la denbufylline.

### Effets sur la protéinurie

L'observation des paramètres cliniques révèle que l'administration du composé NCS 613 jusqu'à la 13^{ème} semaine permet de retarder l'apparition de la protéinurie. A 14 semaines, la protéinurie n'est détectée que chez 10% des souris traitées par le composé NCS 613, contre 70% des souris contrôles. De plus, au delà de la fin du traitement la protéinurie est toujours diminuée chez les souris traitées par le composé NCS 613. En effet, à 20 semaines, la protéinurie n'est détectée que chez 50% des souris traitées par le composé NCS 613, contre 80% des souris contrôles non traitées (voir la Figure 1).

Ainsi, pendant la phase de traitement par le composé NCS613 (jusqu'à 13 semaines), les souris traitées sont protégées de l'apparition de la protéinurie, tandis qu'une protéinurie est détectée chez certaines souris contrôles non traitées dès 10 semaines. Après la fin du traitement par le composé NCS 613, le nombre de souris chez qui une protéinurie est détectée reste inférieur jusqu'à 26 semaines, soit 13 semaines après la fin du traitement.

En administration chronique, le composé NCS 613 semble donc permettre d'empêcher l'apparition de la protéinurie chez les souris MRL/lpr.

En revanche, les résultats obtenus avec la denbufylline, un autre inhibiteur de PDE4 dérivé de l'adénine de structure chimique différente, montrent que la denbufylline ne permet pas de retarder significativement l'apparition de la protéinurie, puisque, contrairement aux souris traitées avec le composé NCS 613, les souris traitées avec la denbufylline présentent à 20 semaines une protéinurie équivalente à celle des souris contrôles.

Ainsi, contrairement à la denbufylline, un autre inhibiteur de PDE4, le composé NCS 613 permet de diminuer significativement la protéinurie de souris MRL/lpr, le développement de la protéinurie étant un facteur clé dans l'évolution de la maladie très polymorphe qu'est le LED.

### Effet sur la durée de vie

Les résultats obtenus montrent que l'administration du NCS 613 induit un effet bénéfique sur la survie des souris MRL /lpr.

En effet, le composé NCS 613 prolonge la durée de vie des animaux de manière sensible à 20 semaines (p=0,0581), cet effet se prolongeant de façon moindre à 26 semaines (p=0,0881 ; voir Figure 2A). Il permet d'augmenter la demi-vie des animaux auto-immuns de manière importante puisqu'il n'y a plus que 30 % de mortalité à 21 semaines au lieu de 60 % de mortalité dans le groupe contrôle non traité (voir Figure 2A).

En revanche, la denbufylline n'a pas d'effet notoire sur la survie des animaux, la mortalité commençant dès 15 semaines et étant quasi identique à celle des animaux contrôles à 26 semaines (p=0,27, voir Figure 2B).

### Effet sur la sécrétion de TNF-α

L'inhibition *ex vivo* de la sécrétion du TNF-α a été mesurée après stimulation des cellules issues du sang de souris (regroupant pour chaque condition le sang de 7 souris) par des lipopolysaccharides bactériens (LPS). Cette expérience a été réalisée sur des souris de différents âges (11 et 14 semaines, soit 2 semaines après la 3^{ème} administration et 1 semaine après la 4^{ème} administration respectivement).

Les résultats montrent que les cellules issues du sang de souris ayant reçu le composé NCS 613 produisent moins de TNF-α en réponse au LPS que les cellules issues du sang des souris du groupe contrôle non traité (voir Figure 3). Le pourcentage d'inhibition de la sécrétion de TNF-α des cellules issues du sang de souris traitées par le composé NCS 613 par rapport aux cellules issues du sang des souris du groupe contrôle PBS est de 44% à 11 semaines, et 54% à 14 semaines (voir Figure 3).

Des résultats équivalents sont obtenus avec la denbufylline (voir Figure 3). A 14 semaines, l'inhibition obtenue avec la denbufylline est même supérieure à celle obtenue avec le composé NCS 613.

### Effet sur la production d'anticorps anti-ADN double brin, marqueurs du lupus

Les résultats (voir Figure 4) montrent que la présence d'Ac anti-ADN sériques apparaît dès l'âge de 11 semaines et est détectable dans pratiquement toutes les souris lupiques non traitées (contrôles) à l'âge de 20 semaines. Le composé NCS 613 retarde l'apparition de ces Ac à l'âge de 20 semaines, qui n'apparaissent en outre que dans un plus petit nombre de souris (20%). A 26 semaines, soit 13 semaines après la fin du traitement, seulement 50% des souris traitées par le composé NCS 613 sont positives pour la présence d'Ac anti-ADN sériques, contre 90% dans le groupe contrôle.

Bien que la denbufylline permette de réduire le pourcentage de souris positives, ses effets sont beaucoup moins importants que le composé NCS 613. En effet, les premières souris positives apparaissent dès 14 semaines (1 semaine seulement après l'arrêt du traitement) au lieu de 20 semaines pour le composé NCS 613. En outre, à 26 semaines, le pourcentage de souris positives reste inférieur dans le groupe traité par le composé NCS 613.

La présence d'Ac anti-ADN sérique est une manifestation importante dans la maladie très polymorphe qu'est le LED. Sur cet aspect, les résultats obtenus indiquent clairement une supériorité du composé NCS 613 par rapport à la denbufylline, autre inhibiteur de PDE4 dérivé de l'adénine.

### Conclusion

L'ensemble de ces résultats démontre que l'administration du composé NCS 613 à des souris pré-autoimmunes MRL/lpr permet de diminuer la capacité des cellules sanguines à sécréter du TNF-α en réponse à une stimulation mitogène d'origine bactérienne et de ralentir la progression de la maladie chez les souris traitées en diminuant la protéinurie et la production d'Ac anti-ADN.

Ainsi, le composé NCS 613 permet d'agir efficacement sur l'ensemble des composantes de la maladie très polymorphe qu'est le LED.

Au contraire, la denbufylline (100µg), autre inhibiteur de PDE4 dérivé de l'adénine, ne possède un effet équivalent au composé NCS 613 (30 µg) que sur l'inhibition de la sécrétion de TNF-α en réponse à une stimulation par le LPS, mais possède des effets beaucoup plus faibles voire quasi inexistants sur les autres composantes de la maladie telles que la protéinurie et la production d'anticorps anti-ADN, ce qui conduit à un effet beaucoup plus faible sur la survie des souris MRL/lpr.

Au total, ces résultats montrent donc que les inventeurs ont mis en évidence une classe de composés particulièrement utiles pour le traitement in vivo du LED puisque permettant de lutter contre l'ensemble des composantes de cette maladie.

### 1.2.3 Effet du composé NCS 613 sur la sécrétion de TNF-α après stimulation des cellules mononucléées du sang de patients auto-immuns par le LPS

Des cellules mononuclées du sang de patients souffrant de maladies auto-immunes systémiques incluant le lupus (LED), la polyarthrite rhumatoïde (PR) et le syndrome de Gougerot-Sjôgren (Goug) ont été incubées ou non en présence du composé NCS 613 puis stimulées par le LPS, et la sécrétion de TNF-α a été mesurée.

Les résultats (voir Figure 5) montrent clairement que le composé NCS 613 utilisé à une concentration de 10µM, inhibe de façon très significative (de 70 à 98% environ) la production de TNF-α par les cellules issues du sang des patients auto-immuns, stimulées par les lipopolysaccharides bactériens (LPS). En particulier, le pourcentage d'inhibition de la sécrétion de TNF-α par les cellules mononuclées du sang de patients souffrant de lupus (LED) est de 69%, 91%, et 98% (86% en moyenne).

Ce résultat obtenu chez l'homme est en accord avec ceux obtenus dans un modèle murin du lupus et montre que le composé NCS 613 est capable d'inhiber la capacité des cellules mononuclées du sang de patients lupiques stimulées par du LPS à sécréter la cytokine inflammatoire TNF-α caractéristique du syndrome inflammatoire induit par le LED.

### 1.2.4 Effet du composé NCS 613 sur la sécrétion basale de TNF-α par cellules mononucléées du sang d'un patient atteint de lupus

Parmi les 3 patients lupiques étudiés, l'un présentait une sécrétion basale de TNF-α, en absence de stimulation par le LPS. La capacité du composé NCS 613 à inhiber cette sécrétion basale de TNF-α a alors été étudiée à partir des cellules issues du sang de ce patient.

Les résultats présentés sur la Figure 6 montrent que le composé NCS 613 permet d'inhiber de 67% la sécrétion spontanée de TNF-α par les cellules issues du sang d'un patient lupique.

Le composé NCS 613 apparaît donc capable d'inhiber efficacement la sécrétion de TNF-α par les cellules mononuclées du sang de patients atteints de LED.

### 1.3 Conclusions

L'ensemble des résultats obtenus chez la souris lupique montre sans ambiguïté que le composé NCS 613 prévient le développement du lupus, en retardant et en diminuant la protéinurie et la production d'Ac anti-ADN marqueurs, en diminuant la réponse inflammatoire et en augmentant la survie des animaux traités. Son effet bénéfique se maintient longtemps après l'arrêt du traitement, suggérant qu'un traitement chronique pourrait être encore plus efficace.

Ces résultats sont spécifiques du composé NCS 613 et de ses dérivés. En effet, l'utilisation d'un autre inhibiteur de PDE4 dérivé de l'adénine, la denbufylline, n'a pas permis d'obtenir des résultats comparables. Ce composé, bien que capable d'inhiber la sécrétion de TNF-α induite par le LPS, n'est pas capable d'avoir un effet comparable au composé NCS 613 sur l'inhibition de la protéinurie et de la production d'anticorps anti-ADN. Les inventeurs ont donc mis en évidence une classe de composés particulièrement efficaces pour le traitement in vivo du LED.

En outre, l'effet du NCS 613 sur la réponse inflammatoire de patients atteints de lupus comme ceux atteints des maladies auto-immunes systémiques telles que la polyarthrite rhumatoïde et le syndrome de Gougerot-Sjögren suggère que le composé NCS 613 est potentiellement capable de traiter le lupus et d'autres maladies auto-immunes.

### EXEMPLE 2 : Corrélation entre l'activité PDE4 au niveau du rein et le développement du lupus chez les souris MRL/Ipr.

### 2.1 Matériels et méthodes

Après sacrifice des souris pré-lupiques (8 semaines, n=3) et lupiques (18 semaines, n=3), les reins ont été prélevés et congelés dans l'azote liquide puis conservés à -80°C jusqu'à la détermination des activités enzymatiques. Les reins sont alors broyés individuellement sous azote liquide, à l'aide d'un cryobroyeur, puis homogénéisés dans un tampon pH 7,5 contenant un cocktail d'inhibiteurs de protéase. Le contenu en protéines est déterminé par la méthode de Lowry et l'activité hydrolytique des PDEs à l'aide d'une méthode radioenzymatique, individuellement pour chaque organe de souris.

Le dosage mesure la transformation de l'AMPc ou du GMPc tritiés respectivement en adénosine ou guanosine tritiés, après addition d'un excès de 5'nucléotidase. Les nucléosides tritiées formées sont séparées des nucléotides cycliques par chromatographie échangeuse d'ions et quantifiés par scintillation liquide.

La contribution de la PDE4 à l'activité hydrolytique totale vis-à-vis de l'AMPc est déterminée en effectuant le dosage en présence ou non de 10 µM de rolipram, inhibiteur spécifique de la PDE4 (Keravis et coll., 2005).

### 2.2. Résultats

Au cours de l'installation du lupus, il y a une augmentation de l'activité de l'hydrolyse de l'AMPc sans modification de l'activité hydrolytique vis-à-vis du GMPc, augurant que seules les enzymes hydrolysant spécifiquement l'AMPc sont modifiées au cours du lupus. De plus cette augmentation est spécifiquement associée à une augmentation d'activité de la PDE4, qui contribue à plus de 50% à l'activité de l'hydrolyse de l'AMPc.

Ainsi, ces résultats montrent pour la première fois une véritable corrélation entre une augmentation de l'activité PDE4 au niveau du rein et le développement de la maladie lupique. L'homme de l'Art ne pouvait pas anticiper ce résultat précis

### REFERENCES

■ Bourguignon, J.J et al., J. Med. Chem. (1997) 40, 1768-1770.
■ Raboisson P. et al., Eur. J. Med. Chem. (2003) 38,199-214).
■ Pharmaceutical salts", J Pharm. Sci. 66:1 (1977).
■ Monneaux F. et al., Eur. J. Immunol. (2003) 33, 287-296.
■ Monneaux, F. et al., . Arthritis Rheum (2004) 50, 3232-3238.
■ Lugnier C. et al., Biochem. Pharmacol. (1986) 35, 1743-1751.
■ Keravis T et al., Meth. Mol. Biol. (2005) 307, 63-74.
■ Kameni Tcheudji J.F et al., J. Mol. Biol. (2001) 310, 181-791.

## Revendications

1. Composé de formule (I) 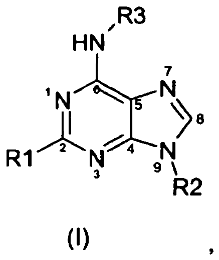 où
R1 est choisi parmi CF₃, un alkyle en C₁-C₅, ou (CH₂)ₙR4 où n est de 0 à 4;
R2 est choisi parmi (CH₂)ₘR4 ou (CH₂)mAr où m est de 0 à 5;
R3 est choisi parmi un hydrogène ou un méthyle ;
R4 est choisi parmi un phényle, OH, un alcoxy en C₁- C₃, un dialkylamino en C₁- C₃, pipéridino, ou N-méthylpipérazino ;
Ar représente 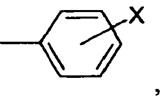 où X est choisi parmi F, Cl, , un alcoxy en C₁- C₃, ou CF₃; ou d'un sel pharmaceutiquement acceptable, d'un énantiomère ou d'un diastéréoisomère de celui-ci, ou d'un mélange de ceux-ci pour son utilisation dans le traitement du lupus érythémateux disséminé.

2. Composé pour son utilisation selon la revendication 1, **caractérisée en ce que** R3 est un méthyle.

3. Composé pour son utilisation selon la revendication 1 ou 2, **caractérisée en ce que** R2 représente (CH₂)Ar, où Ar est tel que défini dans la revendication 1.

4. Composé pour son utilisation selon la revendication 3, **caractérisée en ce que** le substituant X du groupe Ar se trouve en position 2 du cycle benzénique.

5. Composé pour son utilisation selon la revendication 4, **caractérisée en ce que** le substituant X du groupe Ar est choisi parmi un atome de fluor ou un méthoxy.

6. Composé pour son utilisation selon la revendication 5, **caractérisée en ce que** le composé est choisi parmi la 9-(2-fluorobenzyl)-N(6)-méthyl-2-trifluorométhyladénine, la 9-(2-méthoxybenzyl)-N(6)-méthyl-2-n-propyladénine, la 9-(2- méthoxybenzyl)-N(6)-méthyl-2-trifluorométhyladénine, et la 9-(2-méthoxybenzyl)-N(6)- méthyl-2-méthyladénine.

7. Composé pour son utilisation selon la revendication 6, **caractérisée en ce que** le composé est la 9-(2-fluorobenzyl)-N(6)-méthyl-2-trifluorométhyladénine.

8. Composé pour son utilisation tel que défini dans l'une quelconque des revendications 1-7, en association avec un deuxième composé choisi parmi les anti-inflammatoires non stéroïdiens, les anti-paludéens de synthèse, les corticoïdes ou les immunosuppresseurs, pour la fabrication d'un médicament destiné au traitement du lupus érythémateux disséminé, pour une administration simultanée, séparée, étalée dans le temps, ou alternée.

9. Composé pour son utilisation selon la revendication 8, **caractérisée en ce que** le deuxième composé utile pour le traitement du lupus est choisi parmi l'aspirine, l'ibuprofène, l'indométacine, l'hydroxychloroquine, la prednisone, le cyclophosphamide, ou l'azathioprine.

## Patentansprüche

1. Verbindung der Formel (I) 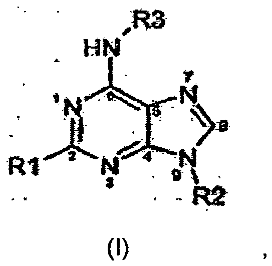 worin:
R1 unter CF₃, einem C₁-C₅-Alkyl oder (CH₂)ₙR4, worin n 0 bis 4 ist, ausgewählt ist;
R2 unter (CH₂)ₘR4 oder (CH₂)ₘAr, worin m 0 bis 5 ist, ausgewählt ist;
R3 unter einem Wasserstoff oder einem Methyl ausgewählt ist;
R4 unter einem Phenyl, OH, einem C₁-C₃-Alkoxy, einem C₁-C₃-Dialkylamino, Piperidino oder N-Methylpiperazino ausgewählt ist;
Ar für 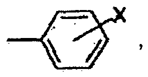 worin X unter F, Cl, einem C₁-C₃-Alkoxy oder CF₃ ausgewählt ist, steht;
oder eins pharmazeutisch annehmbares Salz, ein Enantiomer oder ein Diastereoisomer von jener oder eine Mischung von jenen zu deren Verwendung bei der Behandlung von disseminiertem Lupus erythematodes.

2. Verbindung zu deren Verwendung nach Anspruch 1, **dadurch gekennzeichnet, dass** R3 ein Methyl ist.

3. Verbindung zu deren Verwendung nach Anspruch 1 oder 2, **dadurch gekennzeichnet, dass** R2 für (CH₂)Ar, worin Ar wie in Anspruch 1 definiert ist, steht.

4. Verbindung zu deren Verwendung nach Anspruch 3, **dadurch gekennzeichnet, dass** der Substituent X der Gruppe Ar sich an Position 2 des Benzolrings befindet.

5. Verbindung zu deren Verwendung nach Anspruch 4, **dadurch gekennzeichnet, dass** der Substituent X der Gruppe Ar unter einem Fluoratom oder einem Methoxy ausgewählt ist.

6. Verbindung zu deren Verwendung nach Anspruch 5, **dadurch gekennzeichnet, dass** die Verbindung unter 9-(2-Fluorbenzyl)-N(6)-methyl-2-trifluormethyladenin, 9-(2-Methoxybenzyl)-N(6)-methyl-2-n-propyladenin, 9-(2-Methoxybenzyl)-N(6)-methyl-2-trifluormethyladenin und 9-(2-Methoxybenzyl)-N(6)-methyl-2-methyladenin ausgewählt ist.

7. Verbindung zu deren Verwendung nach Anspruch 6, **dadurch gekennzeichnet, dass** die Verbindung 9-(2-Fluorbenzyl)-N(6)-methyl-2-trifluormethyladenin ist.

8. Verbindung zu deren Verwendung, wie in einem der Ansprüche 1-7 definiert, in Verbindung mit einer zweiten Verbindung, die unter den nicht-steroidalen entzündungshemmenden Mitteln, den synthetischen Malariamitteln, den Kortikoiden oder den Immunsuppressiva ausgewählt ist, für die Herstellung eines Arzneimittels, das für die Behandlung von disseminiertem Lupus erythematodes bestimmt ist, für eine gleichzeitige, getrennte, zeitlich gestaffelte oder alternierende Verabreichung.

9. Verbindung zu deren Verwendung nach Anspruch 8, **dadurch gekennzeichnet, dass** die zweite Verbindung, die für die Behandlung von Lupus nützlich ist, unter Aspirin, Ibuprofen, Indometacin, Hydroxychloroquin, Prednison, Cyclophosphamid oder Azathioprin ausgewählt ist.

## Claims

1. A compound of formula (I) 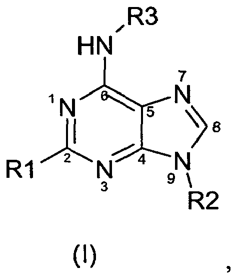 wherein
R1 is selected from CF₃, a C₁-C₅ alkyl, or (CH₂)ₙR4 wherein n is between 0 and 4;
R2 is selected from (CH₂)ₘR4 or (CH₂)ₘAr wherein m is between 0 and 5;
R3 is selected from hydrogen or methyl;
R4 is selected from phenyl, OH, a C₁-C₃ alkoxy, a C₁-C₃ dialkylamino, piperidino, or N-methylpiperazino;
Ar represents 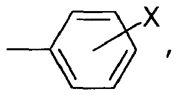 wherein X is selected
from F, Cl, a C₁-C₃ alkoxy, or CF₃;
or a pharmaceutically acceptable salt, an enantiomer or a diastereoisomer thereof, or a mixture thereof, for use in the treatment of systemic lupus erythematosus.

2. A compound for use according to claim 1, **characterised in that** R3 is methyl.

3. A compound for use according to claim 1 or 2, **characterised in that** R2 represents (CH₂)Ar, wherein Ar is as defined in claim 1.

4. A compound for use according to claim 3, **characterised in that** the X substituent of the Ar group is in position 2 of the benzene ring.

5. A compound for use according to claim 4, **characterised in that** the X substituent of the Ar group is selected from a fluorine atom or a methoxy.

6. A compound for use according to claim 5, **characterised in that** the compound is selected from 9-(2-fluorobenzyl)-N(6)-methyl-2-trifluoromethyladenine, 9-(2-methoxybenzyl)-N(6)-methyl-2-n-propyladenine, 9-(2-methoxybenzyl)-N(6)-methyl-2-trifluoromethyladenine, and 9-(2-methoxybenzyl)-N(6)-methyl-2-methyladenine.

7. A compound for use according to claim 6, **characterised in that** the compound is 9-(2-fluorobenzyl)-N(6)-methyl-2-trifluoromethyladenine.

8. A compound for use as defined in any one of claims 1-7, in combination with a second compound selected from non-steroid anti-inflammatory drugs, synthetic antimalarials, corticosteroids or immunosuppressants, for the manufacture of a drug intended for the treatment of systemic lupus erythematosus, to be administered simultaneously, separately, spread over a period of time, or alternately.

9. A compound for use according to claim 8, **characterised in that** the second compound useful for the treatment of lupus is selected from aspirin, ibuprofen, indomethacin, hydroxychloroquine, prednisone, cyclophosphamide, or azathioprine.
